# FASCICULE DE BREVET EUROPEEN

(11) **EP 1 691 808 B1**
(45) Date de publication et mention de la délivrance du brevet: **22.04.2009**
(21) Numéro de dépôt: 04805418.3
(22) Date de dépôt: 09.11.2004
(51) Int. Cl.: A61K 31/454, A61K 9/08, A61K 9/10

(54) **COMPOSITION PHARMACEUTIQUE DESTINEE A L'ADMINISTRATION ORALE D'UN DERIVE DE PYRAZOLE-3-CARBOXAMIDE**
PHARMAZEUTISCHE ZUSAMMENSETZUNG FÜR DIE ORALE VERABREICHUNG EINES PYRAZOL-3-CARBONSÄUREDERIVATS
PHARMACEUTICAL COMPOSITION FOR ORAL ADMINISTRATION OF A PYRAZOL-3-CARBOXAMIDE DERIVATIVE

(30) Priorité: 10.11.2003 FR 0313259
(43) Date de publication de la demande: 23.08.2006
(73) Titulaire: Sanofi-Aventis, 75013 Paris (FR)
(72) Inventeur: BREUL, Thierry, F-34110 Frontignan (FR); GAUTIER, Jean Claude, FR-34830 Clapiers (FR); SASLAWSKI, Olivier, F-34000 Montpellier (FR)
(74) Mandataire: Kugel, Dominique
(86) Numéro de dépôt international: PCT/FR2004/002875
(87) Numéro de publication internationale: WO 2005/046690

(56) Documents cités:
- EP-A- 0 969 832
- WO-A-20/04009057
- FR-A- 2 761 265
- FR-A- 2 831 883
- US-A- 5 059 691
- US-A- 5 624 941
- US-A1- 2003 003 145

## Description

La présente invention se rapporte à une composition pharmaceutique destinée à l'administration orale d'un dérivé de pyrazole-3-carboxamide ainsi que de ses sels pharmaceutiquement acceptables et leurs solvats.

Par dérivé de pyrazole-3-carboxamide, on entend un composé choisi parmi le N-pipéridino-5-(4-bromophényl)-1-(2,4-dichlorophényl)-4-éthylpyrazole-3-carboxamide et le N-pipéridino-5-(4-chlorophényl)-1-(2,4-dichlorophényl)-4-méthylpyrazole-3-carboxamide. Dans la présente description, ces composés sont nommés comme "principe actifs selon l'invention".

Le N-pipéridino-5-(4-bromophényl)-1-(2,4-dichlorophényl)-4-éthylpyrazole-3-carboxamide, ci-après dénommé composé A, est décrit dans le brevet européen EP-B-1150961. Le N-pipéridino-5-(4-chlorophényl)-1-(2,4-dichlorophényl)-4-méthyl pyrazole-3-carboxamide, ci-après dénommé composé B, dont la dénomination commune internationale est rimonobant, est décrit dans le brevet européen EP-B-656354. Ces composés sont des antagonistes des récepteurs CB₁ aux cannabinoïdes.

Ces composés sont des molécules très peu solubles dans l'eau, respectivement : 0,1 µg/ml et 1 µg/l, à pH = 6,5. De plus, ces composés ont des coefficients de perméabilité membranaire élevés : respectivement 78.10⁻⁷ cm/s et 96.10⁻⁷ cm/s sur le modèle de cellules CaCO₂, tel que décrit par M. C. Gres et al. dans Pharmaceutical Research, 1198, 15(5), 726-7333.

Une composition pharmaceutique contenant un dérivé de pyrazole-3-carboxamide sous forme micronisée et un agent mouillant tensioactif a été décrite dans le brevet européen EP-B-969832. Une composition pharmaceutique contenant le composé B en mélange avec le Poloxamer 127 et un macrogolglyrécide est décrite dans la demande internationale WO 98/43635.

La demande de brevet WO 2004/009057 décrit un procédé pour préparer une dispersion de nanoparticules cristallines dans un milieu aqueux et l'utilisation de tensioactif à une concentration faible permettant d'éviter la solubilisation desdites nanoparticules ; des exemples de réalisation concernent notamment le composé A et le composé B.

On a maintenant trouvé des compositions pharmaceutiques contenant un dérivé de pyrazole-3-carboxamide selon l'invention qui permettent d'améliorer la solubilisation des principes actifs selon l'invention et la biodisponibilité chez l'homme à l'état à jeun.

Ces compositions pharmaceutiques sont constituées d'un mélange homogène, hydrodispersible, dans lequel le principe actif selon l'invention est solubilisé dans un solvant lipidique auquel on ajoute un agent tensioactif hydrophile afin de former spontanément une émulsion fine ou une microémulsion lors de leur dilution en milieu aqueux, ces compositions sont dites auto-émulsionnables ou auto-microémulsionnables.

Une microémulsion est un système transparent thermodynamiquement stable (Microemulsion and related system in Surfactant Sciences Series, Marcel Dekker Inc., 1988, 30, p. 25-26).

Par émulsion fine, on entend une émulsion dans laquelle la taille des globules dispersés est inférieure à 5 µm. Cette émulsion fine est caractérisée par le fait qu'elle est suffisamment stable pour perdurer dans le tractus gastro-intestinal jusqu'au site d'absorption, c'est à dire l'intestin.

Ainsi, la présente invention est relative à une composition pharmaceutique sous forme liquide ou semi-solide, auto-émulsionnable ou auto-microémulsionnable en milieu aqueux, pour l'administration orale d'un dérivé de pyrazole-3-carboxamide choisi parmi : le N-pipéridino-5-(4-bromophényl)-1-(2,4-dichlorophényl)-4-éthylpyrazole-3-carboxamide et le N-pipéridino-5-(4-chlorophényl)-1-(2,4-dichlorophényl)-4-méthylpyrazole-3-carboxamide, dans laquelle ledit dérivé de pyrazole-3-carboxamide est solubilisé dans un mélange contenant un ou plusieurs solvants lipidiques du dérivé pyrazole-3-carboxamide et un agent tensioactif hydrophile non ionique dont la balance hydrophile-lipophile est supérieure à 10 et préférentiellement comprise entre 10 et 18.

Selon la présente invention, la proportion pondérale du principe actif est comprise entre 0,1 et 6 %, préférentiellement entre 0,1 et 5%.

Dans la composition pharmaceutique selon l'invention, la proportion pondérale du solvant lipidique ou du mélange de solvants lipidiques est de 35 à 75 %, préférentiellement 35 à 55 %.

Préférentiellement le mélange de la composition pharmaceutique selon l'invention contient également un cosolvant amphiphile ou un mélange de cosolvants amphiphiles. La présence d'un tel cosolvant amphiphile favorise la solubilisation du principe actif selon l'invention et l'émulsification ultérieure de la composition pharmaceutique en milieu aqueux. Lorsqu'il est présent, le cosolvant amphiphile, ou chacun des cosolvants amphiphiles, est dans une proportion pondérale inférieure à 30%. Lorsque 2 solvants amphiphiles sont présents, ils sont dans une proportion pondérale totale inférieure à 50 %, préférentiellement inférieure à 45 %.

Ainsi, la composition pharmaceutique selon la présente invention contient préférentiellement de 10 à 50 % de cosolvant(s) amphiphile(s), plus particulièrement de 10 à 45 %.

Préférentiellement, l'agent tensioactif hydrophile non ionique est constitué soit- d'un seul tensioactif dont la balance hydrophile-lipophile est supérieure à 10, soit d'un mélange de tensioactifs, la balance hydrophile-lipophile dudit mélange étant supérieure à 10. Selon la présente invention, l'agent tensioactif est dans une proportion pondérale de 5 à 50%, préférentiellement de 5 à 25% et de manière optimale de 5 à 15%.

Ainsi la concentration d'agent tensioactif utilisée selon la présente invention est nettement supérieure à la concentration micellaire critique (CMC), de manière à mettre en oeuvre la capacité de solubilisation dudit agent tensioactif dans les conditions de la présente invention.

Les compositions pharmaceutiques selon la présente invention, peuvent être administrées dans des capsules de gélatine molles ou dans des capsules de gélatine dures scellées ou banderolées.

Selon la présente invention, on peut utiliser les tensioactifs non ioniques tels que :
Polyoxyéthylène 35 huile de ricin hydrogénée : Cremophor^{®} EL, Polyoxyéthylène 40 huile de ricin hydrogénée : Cremophor^{®} RH40, tous les deux commercialisés par BASF.
Polyoxyéthylène polysorbate: Tween^{®} 80, Tween^{®} 20, Tween^{®} 60, Tween^{®} 85, commercialisés par ICI.
Sorbitan mono laurate : Span 20, Sorbitan monooléate : Span 80, tous les deux commercialisés par ICI.
Vitamine E/TPGS : Tocopherol propylène glycol 1000 succinate, commercialisé par Eastman.
Hydroxystéarate de polyéthylène glycol 15 : Solutol^{®} HS15, commercialisé par BASF.

Les tensioactifs hydrophiles préférés, seuls ou en mélange, sont le Cremophor^{®} RH40, Cremophor^{®} EL, la vitamine E TPGS, le Tween 80.

Les tensioactifs tels que les Span étant lipophiles sont utilisés en mélange avec d'autres tensioactifs de manière que la balance hydrophile-lipohile du mélange des tensioactifs soit supérieure à 10.

Par solvants lipidiques, et cosolvant amphiphiles, on entend des dérivés d'acides gras naturels, préférentiellement d'origine végétale, obtenus par estérification avec un alcool :
- soit le glycérol (mono-, di-, tri-glycérides),
- soit un glycol, éventuellement à longue chaîne (macrogolglycérides).

Selon la longueur de la chaîne de l'acide gras et selon la nature de l'alcool, ces solvants présentent un caractère plus ou moins amphiphile.

Selon la présente invention, on peut utiliser des solvants lipidiques tels que :
Oleoyl macrogol 6 glycérides (glycérides insaturés polyglycosylés) : Labrafil^{®} 1944 CS, commercialisé par Gattefossé.
Propylène glycol caprylate caprate : Labrafac^{®} PG, commercialisé par Gattefossé.
Propylène glycol caprylic acid mono ester : Capmul^{®} PG-8, commercialisé par Abitec.
Glycéryloléate : Peceol^{®} commercialisé par Gattefossé.
Mono et diglycéride à chaine moyenne (capric caprylique) : Capmul^{®} MCM, commercialisé par Abitec.
Polyglycérol oléate : Plurol^{®} oléique, commercialisé par Gattefossé.
Caprylic/capric triglycéride : Miglyol^{®} 812, commercialisé par Dynamit Nobel, Labrafac^{®} CC, commercialisé par Gattefossé.

Les solvants de lipidiques préférés, seuls ou en mélange, sont le Labrafil^{®} 1944 CS et le Miglyol^{®} 812 ou le Labrafac^{®} CC ou le Capmul^{®} MCM.

Selon la présente invention, on peut utiliser des cosolvants amphiphiles tels que :
Propylène glycol monolaurate : Capmul^{®} PG12, commercialisé par Abitec.
Propylène glycol monolaurate : Lauroglycol^{®} 90, commercialisé par Gattefossé.
Caprylocaproyl macrogol 8 glycérides : (glycérides saturés éthyldiglycosilés) : Labrasol^{®}, Gélucire 44-14 commercialisé par Gattefossé, le Diéthylène glycol mono ethyl ether : Transcutol^{®}, commercialisé par Gattefossé.
PEG 400 : Polyéthylène glycol 400, commercialisé par Huls ou ICI.

Les solvants amphiphiles préférés, seuls ou en mélange, sont le Labrasol et le Gélucire 44-14, le Capmul^{®} PG12 ou le Lauroglycol^{®} 90.

### EXEMPLES

On prépare plusieurs compositions pharmaceutiques selon l'invention en utilisant le mode opératoire suivant : on mélange le (ou les) solvant(s) lipidique(s) choisis ainsi que l'agent tensioactif à une température comprise entre 30 et 65°C, préférentiellement entre 40 et 45°C, sous agitation ; et ce après avoir fondu les différents solvants si nécessaire. On incorpore le principe actif en maintenant l'agitation le temps nécessaire à la solubilisation dudit principe actif puis on transfère la formulation ainsi obtenue vers le poste de mise en gélules.

### EXEMPLE 1 : Formulations avec solvant(s) lipidique(s) et tensioactif.

| Formulation Exemple | Composants | % pondéral |
|---|---|---|
| 1.1 | Miglyol 812 | 49,6 |
| | Vitamine E TPGS | 49,6 |
| | Composé A | 0,8 |

| Formulation Exemple | Composants | % pondéral |
|---|---|---|
| 1.2 | Miglyol 812 | 74,4 |
| | Vitamine E TPGS | 24,8 |
| | Composé A | 0,8 |

### EXEMPLE 2 : Formulations avec solvant lipidique, tensioactif et cosolvant(s) amphiphile(s).

| Formulation Exemple | Composants | % pondéral |
|---|---|---|
| 2.1 | Miglyol 812 | 45,9 |
| | Crémophor RH 40 | 12 |
| | Lauroglycol 90 | 21,5 |
| | Labrasol | 20 |
| | Composé A | 0,6 |

| Formulation Exemple | Composants | % pondéral |
|---|---|---|
| 2.2 | Miglyol 812 | 41,5 |
| | Crémophor RH 40 | 12 |
| | Labrasol | 20 |
| | Lauroglycol 90 | 21,5 |
| | Composé B | 5 |

| Formulation Exemple | Composants | % pondéral |
|---|---|---|
| 2.3 | Miglyol 812 | 45,25 |
| | Vitamine E TPGS | 12 |
| | Labrasol | 20 |
| | Lauroglycol 90 | 21,5 |
| | Composé A | 1,25 |

| Formulation Exemple | Composants | % pondéral |
|---|---|---|
| 2.4 | Labrafil 1944 CS | 53,8 |
| | Tween 80 | 9,8 |
| | Labrasol | 17,9 |
| | Lauroglycol 90 | 17,9 |
| | Composé A | 0,6 |

| Formulation Exemple | Composants | % pondéral |
|---|---|---|
| 2.5 | Labrafil 1944 CS | 70 |
| | Crémophor RH 40 | 9,5 |
| | Span 20 | 2,4 |
| | Labrasol | 17,5 |
| | Composé A | 0,6 |

| Formulation Exemple | Composants | % pondéral |
|---|---|---|
| 2.6 | Labrafil 1944 CS | 39,8 |
| | Tween 85 | 49,7 |
| | Labrasol | 10 |
| | Composé B | 0,5 |

| Formulation Exemple | Composants | % pondéral |
|---|---|---|
| 2.7 | Miglyol 812 | 41,5 |
| | Crémophor RH 40 | 12 |
| | Labrasol | 20 |
| | Lauroglycol 90 | 21,5 |
| | Composé A | 5 |

| Formulation Exemple | Composants | % pondéral |
|---|---|---|
| 2.8 | Labrafac CC | 45,9 |
| | Crémophor EL | 12 |
| | Labrasol | 20 |
| | Lauroglycol 90 | 21,5 |
| | Composé A | 0,6 |

| Formulation Exemple | Composants | % pondéral |
|---|---|---|
| 2.9 | Miglyol812 | 41,5 |
| | Crémophor RH 40 | 12 |
| | Gélucire 44-14 | 20 |
| | Lauroglycol 90 | 21,5 |
| | Composé B | 5 |

| Formulation Exemple | Composants | % pondéral |
|---|---|---|
| 2.10 | Miglyol 812 | 41,5 |
| | Crémophor RH 40 | 12 |
| | Labrasol | 20 |
| | Lauroglycol 90 | 11 |
| | Capmul MCM | 10,5 |
| | Composé B | 5 |

| Formulation Exemple | Composants | % pondéral |
|---|---|---|
| 2.11 | Miglyol 812 | 41,5 |
| | Crémophor RH 40 | 12 |
| | Gélucire 44-14 | 20 |
| | Lauroglycol 90 | 21,5 |
| | Composé A | 5 |

| Formulation Exemple | Composants | % pondéral |
|---|---|---|
| 2.12 | Miglyol 812 | 41,5 |
| | Crémophor RH 40 | 12 |
| | Labrasol | 20 |
| | Lauroglycol 90 | 15,5 |
| | Capmul MCM | 6 |
| | Composé B | 5 |

La capacité à former une émulsion fine et stable est évaluée pour chacune des formulations ci-dessus en diluant celles-ci au 1/10^{ème} dans un milieu intestinal simulé de pH 6.

On observe d'une part le temps de début de décantation qui signe la stabilité de l'émulsion et d'autre part, sous microscope, la taille des globules huileux dispersés dans la phase aqueuse pour contrôler sa finesse.

Dans tous les cas, le temps de début de décantation dépasse largement 24 heures.

Les globules qui sont visibles en microscopie optique sont souvent d'un diamètre d'environ 1 micron, les plus gros pouvant atteindre 5 microns.

Mesures des cinétiques de dissolution *in vitro* :

Les cinétiques de dissolution sont étudiées dans l'appareil à pales (appareil n°2 de la Pharmacopée) dans un milieu physiologique simulé de pH 6, à 37°C, et avec une agitation de 75 t/min.

La formulation en gélule est introduite dans l'appareil de dissolution au temps 0 et le pourcentage de produit finement émulsionné est déterminé aux temps 15, 30, 60 minutes puis 2, 3 et 4 heures par dosage HPLC (de l'anglais High Performance Liquid Chromatography = chromatographie liquide à haute performance) du milieu de dissolution, après filtration sur 5 µm. (Ainsi on s'assure bien de ne doser que le principe actif qui se trouve sous forme d'émulsion suffisamment fine pour ne pas être retenu par le filtre de 5 µm).

Le temps de l'expérience est supérieur à celui nécessaire pour atteindre l'intestin (2 à 3 heures) qui est le site principal d'absorption.

A titre de comparaison, on a effectué le même test de dissolution avec une formulation dite de référence, décrite ci-après:

### Formulation de référence :

| Composants | mg/unité |
|---|---|
| Composé A | 10 |
| Amidon de maïs | 80 |
| Lactose monohydrate 200 mesh | 274 |
| Hypromellose | 10 |
| Sodium laurylsulfate | 2 |
| Eau purifiée | QS |
| croscarmellose sodique | 20 |
| Stéarate de magnésium | 4 |
| Gélule taille n° 0 | gélule remplie à 400 mg |

L'expérience est conduite avec la même concentration initiale en composé A dans le milieu pour chaque test de dissolution. Ainsi dans 250 ml de milieu de dissolution, on place soit 1 gélule de référence contenant 10 mg de composé A, soit 10 mg de composé A provenant de gélule préparée à partir soit de la formulation 1.1, soit de la formulation 2.1.

**TABLEAU 1**

| Dissolution *in-vitro* | | | | | | |
|---|---|---|---|---|---|---|
| Temps en minutes | 15 | 30 | 60 | 120 | 180 | 240 |
| % de Composé A en émulsion fine avec la formulation 1.1 | 94,2 | 95,3 | 92,2 | 95 | 92,5 | 91,2 |
| % de composé A en émulsion fine avec la formulation 2.1 | 33,6 | 82,5 | 97 | 99,7 | 100 | 99,6 |
| % de composé A solubilisé avec la formulation de référence | 14,2 | 23 | 23,8 | 26,7 | 20 | 22,7 |

On constate que les formulations selon l'invention permettent de "solubiliser" par l'émulsion fine plus de 80 % du composé selon l'invention en 30 minutes et que cet état solubilisé persiste pendant au moins 4 heures, au contraire, la formulation de référence ne permet de solubiliser qu'environ 25% du principe actif.

Mesures de biodisponibilité chez l'homme :

Les compositions pharmaceutiques selon l'invention ont également été évaluées *in-vivo* chez l'homme afin d'étudier l'influence de la formulation selon l'invention sur la biodisponibilité du principe actif à l'état à jeun et à l'état nourri.

Dans un premier essai, on a comparé pour la formulation de référence décrite ci-dessus, la biodisponibilité du principe actif à jeun versus nourri.

Dans cet essai, une dose de 50 mg de composé A est administrée par voie orale, en une prise, à 12 volontaires sains et les 2 administrations à jeun et nourri sont faites de façon randomisées et à 21 jours d'intervalle.

Des prélèvements sanguins sont effectués après administration aux temps : 30 minutes, 1 heure, 1,5 heure, 2 heures, 2,5 heures, 3 heures, 4 heures, 6 heures, 8 heures, 12 heures, 24 heures, 36 heures, 48 heures, 72 heures, 120 heures et 168 heures. Les différents paramètres pharmacocinétiques permettant d'établir la biodisponibilité du principe actif sont mesurés.

**TABLEAU 2**

| Biodisponibilité du composé A avec la formulation de référence | | | |
|---|---|---|---|
| | Tmax | Cmax | AUC |
| | (heure) | (ng/ml) | (ng-h/ml) |
| Nourri | 4(2,5-6,0) | 524 (152) | 15949 (6192) |
| A jeun | 2,0 (1,0 - 4,0) | 126 (60) | 4480 (1542) |

Avec la formulation de référence, on constate que les valeurs de Cmax et AUC (aire sous la courbe) sont respectivement 4,3 et 3,5 fois plus grandes pour les sujets nourris que pour les sujets à jeun.

Dans un deuxième essai, on a évalué la biodisponibilité du principe actif avec les formulations selon la présente invention.

Dans cet essai, une dose de 10 mg du composé A est administrée à 12 volontaires sains, à jeun, en une prise par voie orale, soit par la formulation de référence, soit par la formulation 1.1 en capsule de gélatine dure, soit par la formulation 2.1 en capsule de gélatine molle. Les administrations sont faites de façon randomisée, à 8 jours d'intervalle. Les prélèvements sanguins sont effectués comme dans l'essai précédent et les paramètres pharamacocinétiques sont mesurés.

Les résultats montrent une amélioration de la biodisponibilité du principe actif à jeun par rapport aux résultats obtenus avec la formulation de référence.

Après un délai de 15 jours, on a administré la formulation 2.1 dans les mêmes conditions, aux mêmes patients nourris et on a mesuré les paramètres pharmacocinétiques.

**TABLEAU 3**

| Absorption du composé A chez des sujets nourris et à jeun | | | | |
|---|---|---|---|---|
| Paramètre | Formulation de référence administrée à jeun | Formulation 2.1 administrée à jeun | Formulation 1.1 administrée à jeun | Formulation 2.1 administrée nourri |
| Cmax ng/ml | 47 (17) | 160 (38) | 147 (33) | 139 (37,7) |
| Tmax heure | 1,5 (1,3) | 1 (1,2) | 1,5 (1,3) | 1,5 (0,5) |
| AUC ng.h/ml | 906 (420) | 1520 (664) | 1350 (497) | 1760 (782) |

| | | | | |
|---|---|---|---|---|
| Les valeurs entre parenthèses ( ) indiquent les déviations standards. | | | | |

Avec les formulations selon l'invention, on constate que les valeurs de Cmax et AUC sont voisines quelque soit la formulation lorsque le sujet est à jeun.

L'amélioration de la biodisponibilité à l'état à jeun, basée sur l'augmentation de l'AUC est respectivement de 165% et 152% pour la formulation 2.1 et 1.1 vis à vis de la formulation de référence.

De plus, avec la formulation 2.1, on voit que la différence de biodisponibilité entre l'état à jeun et l'état nourri n'est plus significative.

Ainsi, les formulations selon l'invention permettent d'améliorer significativement la biodisponibilité à l'état à jeun, conduisant ainsi à supprimer la différence de biodisponibilité entre l'état nourri et l'état à jeun.

## Revendications

1. Composition pharmaceutique sous forme liquide ou semi-solide, auto-émulsionnable ou auto-microémulsionnable en milieu aqueux, pour l'administration orale d'un dérivé de pyrazole-3-carboxamide choisi parmi: le N-pipéridino-5-(4-bromophényl)-1-(2,4-dichlorophényl)-4-éthylpyrazole-3-carboxamide et le N-pipéridino-5-(4-chlorophényl)-1-(2,4-dichlorophényl)-4-méthylpyrazole-3-carboxamide, dans laquelle ledit dérivé de pyrazole-3-carboxamide est solubilisé dans un mélange contenant un ou plusieurs solvants lipidiques du dérivé pyrazole-3-carboxamide dans une proportion pondérale de 35 à 75 %, et un agent tensioactif hydrophile non ionique dont la balance hydrophile-lipophile est comprise entre 10 et 18, dans une proportion pondérale de 5 à 50 %.

2. Composition pharmaceutique selon la revendication 1 contenant en outre un cosolvant amphiphile ou un mélange de cosolvants amphiphiles.

3. Composition pharmaceutique selon la revendication 2 dans laquelle le cosolvant amphiphile ou chacun des cosolvants amphiphiles présents est dans une proportion pondérale inférieure à 30 %.

4. Composition pharmaceutique selon l'une quelconque des revendications 2 ou 3 dans laquelle le cosolvant amphiphile ou le mélange de cosolvants amphiphiles est dans une proportion pondérale comprise entre 10 et 50 %.

5. Composition pharmaceutique selon l'une quelconque des revendications 1 à 4 dans laquelle le solvant lipidique ou le mélange de solvants lipidiques est dans une proportion pondérale de 35 à 55 %.

6. Composition pharmaceutique selon l'une quelconque des revendications 1 à 5 dans laquelle l'agent tensioactif est constitué d'un seul tensioactif ou d'un mélange de tensioactifs.

7. Composition pharmaceutique selon l'une quelconque des revendications 1 à 6 dans laquelle l'agent tensioactif est dans une proportion pondérale de 5 à 15%.

8. Composition pharmaceutique selon l'une quelconque des revendications 1 à 7 dans laquelle le dérivé de pyrazole-3-carboxamide est dans une proportion pondérale de 0,1 à 6%.

9. Composition pharmaceutique selon l'une quelconque des revendications 1 à 8 dans laquelle le ou les solvants lipidiques sont choisis parmi :
. Oleoyl macrogol 6 glycérides (glycérides insaturés polyglycosylés) ;
. Propylène glycol caprylate caprate ;
. Propylène glycol caprylic acid mono ester ;
. Oleoyl macrogol 6 glycérides (glycérides insaturés polyglycosylés) ;
. Propylène glycol caprylate caprate ;
. Propylène glycol caprylic acid mono ester ;
. Glycéryloléate ;
. Mono et diglycéride à chaîne moyenne (capric caprylique) ;
. Polyglycérol oléate ;
. Caprylic/capric triglycéride.

10. Composition selon l'une quelconque des revendications 1 à 8 dans laquelle le ou les tensio-actifs sont choisis parmi :
. Polyoxyéthylène 35 huile de ricin hydrogénée ;
. Polyoxyéthylène 40 huile de ricin hydrogénée ;
. Polyoxyéthylène polysorbate ;
. Sorbitan mono laurate ;
. Vitamine E/TPGS : Tocopherol propylène glycol 1000 succinate ;
. Hydroxystéarate de polyéthylène glycol 15.

11. Composition pharmaceutique selon la revendication 2 dans laquelle le ou les cosolvants amphiphiles sont choisis parmi :
. Caprylocaproyl macrogol 8 glycérides ;
. Propylène glycol monolaurate.

12. Composition pharmaceutique selon l'une quelconque des revendications 1 à 11 administrable dans des capsules de gélatine molles.

13. Composition pharmaceutique selon l'une quelconque des revendications 1 à 11 administrable dans des capsules de gélatine dures scellées ou banderolées.

14. Composition pharmaceutique contenant :
. Caprylic/capric triglycéride 45,9 %
. Polyoxyéthylène 40 huile de ricin hydrogénée 12 %
. Propylène glycol monolaurate 21,5 %
. Caprylocaproyl macrogol 8 glycérides 20 %
. N-pipéridino-5-(4-bromophényl)-1-(2,4-dichlorophényl) -4-éthylpyrazole-3-carboxamide 0,6 %

15. Composition pharmaceutique contenant :
. Caprylic/capric triglycéride 41,5 %
. Polyoxyéthylène 40 huile de ricin hydrogénée 12 %.
. Propylène glycol monolaurate 21,5 %
. Caprylocaproyl macrogol 8 glycérides 20 %
. N-pipéridino-5-(4-bromophényl)-1-(2,4-dichlorophényl)

16. Composition pharmaceutique contenant :
. Caprylic/capric triglycéride 41,5 %
. Polyoxyéthylène 40 huile de ricin hydrogénée 12 %
. Propylène glycol monolaurate 21,5 %
. Caprylocaproyl macrogol 8 glycérides 20 %
. N-pipéridino-5-(4-chlorophényl)-1-(2,4-dichlorophényl)-4-méthylpyrazole-3-carboxamide 5%

## Claims

1. Pharmaceutical composition in liquid or semi-solid form, which is self-emulsifying or self-microemulsifying in aqueous medium, for the oral administration of a pyrazole-3-carboxamide derivative chosen from: N-piperidino-5-(4-bromophenyl)-1-(2,4-dichlorophenyl)-4-ethyl-pyrazole-3-carboxamide and N-piperidino-5-(4-chlorophenyl)-1-(2,4-dichlorophenyl)-4-methylpyrazole-3-carboxamide, in which the said pyrazole-3-carboxamide derivative is dissolved in a mixture containing one or more lipid solvents for the pyrazole-3-carboxamide derivative in a weight proportion of from 35% to 75%, and a nonionic hydrophilic surfactant whose hydrophilic/lipophilic balance is between 10 and 18, in a weight proportion of from 5% to 50%.

2. Pharmaceutical composition according to Claim 1, also containing an amphiphilic cosolvent or a mixture of amphiphilic cosolvents.

3. Pharmaceutical composition according to Claim 2, in which the amphiphilic cosolvent or each of the amphiphilic cosolvents present is in a weight proportion of less than 30%.

4. Pharmaceutical composition according to either of Claims 2 and 3, in which the amphiphilic cosolvent or the mixture of amphiphilic cosolvents is in a weight proportion of between 10% and 50%.

5. Pharmaceutical composition according to any one of Claims 1 to 4, in which the lipid solvent or the mixture of lipid solvents is in a weight proportion of from 35% to 55%.

6. Pharmaceutical composition according to any one of Claims 1 to 5, in which the surfactant consists of a single surfactant or of a mixture of surfactants.

7. Pharmaceutical composition according to any one of Claims 1 to 6, in which the surfactant is in a weight proportion of from 5% to 15%.

8. Pharmaceutical composition according to any one of Claims 1 to 7, in which the pyrazole-3-carboxamide derivative is in a weight proportion of from 0.1% to 6%.

9. Pharmaceutical composition according to any one of Claims 1 to 8, in which the lipid solvent (s) is (are) chosen from:
■ oleoyl macrogol 6 glycerides (unsaturated polyglycosyl glycerides);
■ propylene glycol caprylate caprate;
■ propylene glycol caprylic acid monoester;
■ glyceryl oleate;
■ medium-chain (capric caprylic) mono- and diglyceride;
■ polyglycerol oleate;
■ caprylic/capric triglyceride.

10. Composition according to any of one of Claims 1 to 8, in which the surfactant(s) is (are) chosen from:
■ polyoxyethylene 35 hydrogenated castor oil;
■ polyoxyethylene 40 hydrogenated castor oil;
■ polyoxyethylene polysorbate;
■ sorbitan monolaurate;
■ vitamin E/TPGS: tocopherol propylene glycol 1000 succinate;
■ polyethylene glycol 15 hydroxystearate.

11. Pharmaceutical composition according to Claim 2, in which the amphiphilic cosolvent(s) is (are) chosen from:
■ caprylocaproyl macrogol 8 glycerides;
■ propylene glycol monolaurate.

12. Pharmaceutical composition according to any one of Claims 1 to 11, which may be administered in soft gelatin capsules.

13. Pharmaceutical composition according to any one of Claims 1 to 11, which may be administered in sealed or film-coated hard gelatin capsules.

14. Pharmaceutical composition containing:
■ caprylic/capric triglyceride 45.9%
■ polyoxyethylene 40 hydrogenated 12% castor oil
■ propylene glycol monolaurate 21.5%
■ caprylocaproyl macrogol 8 glycerides 20%
■ N-piperidino-5-(4-bromophenyl)-1- 0.6% (2,4-dichlorophenyl)-4-ethylpyrazole-3-carboxamide

15. Pharmaceutical composition containing:
■ caprylic/capric triglyceride 41.5%
■ polyoxyethylene 40 hydrogenated 12% castor oil
■ propylene glycol monolaurate 21.5%
■ caprylocaproyl macrogol 8 glycerides 20%
■ N-piperidino-5-(4-bromophenyl)-1- 5% (2,4-dichlorophenyl)-4-ethylpyrazole-3-carboxamide

16. Pharmaceutical composition containing:
■ caprylic/capric triglyceride 41.5%
■ polyoxyethylene 40 hydrogenated 12% castor oil
■ propylene glycol monolaurate 21.5%
■ caprylocaproyl macrogol 8 glycerides 20%
■ N-piperidino-5-(4-chlorophenyl)-1- 5% (2,4-dichlorophenyl)-4-methylpyrazole-3-carboxamide

## Patentansprüche

1. Pharmazeutische Zusammensetzung in flüssiger oder halbfester Form, die in wässrigem Medium autoemulgierbar oder auto-mikroemulgierbar ist, zur oralen Verabreichung eines Pyrazol-3-carboxamid-Derivats, das ausgewählt ist aus: N-Piperidino-5-(4-bromphenyl)-1-(2,4-dichlorphenyl)-4-ethylpyrazol-3-carboxamid und N-Piperidino-5-(4-chlorphenyl)-1-(2,4-dichlorphenyl)-4-methylpyrazol-3-carboxamid, wobei das Pyrazol-3-carboxamid-Derivat in einem Gemisch solubilisiert wird, das ein oder mehrere lipidische Lösungsmittel des Pyrazol-3-carboxamid-Derivats in einem Gewichtsanteil von 35 bis 75% und ein hydrophiles, nicht-ionisches oberflächenaktives Mittel mit einer Hydrophilen-Lipophilen-Balance zwischen 10 und 18 in einem Gewichtsanteil von 5 bis 50% enthält.

2. Pharmazeutische Zusammensetzung nach Anspruch 1, die außerdem ein amphiphiles Co-Lösungsmittel oder ein Gemisch von amphiphilen Co-Lösungsmitteln enthält.

3. Pharmazeutische Zusammensetzung nach Anspruch 2, wobei das amphiphile Co-Lösungsmittel oder jedes der vorhandenen amphiphilen Co-Lösungsmittel in einem Gewichtsanteil von weniger als 30% vorliegt.

4. Pharmazeutische Zusammensetzung nach einem der Ansprüche 2 oder 3, wobei das amphiphile Co-Lösungsmittel oder das Gemisch von amphiphilen Co-Lösungsmitteln in einem Gewichtsanteil zwischen 10 und 50% vorliegt.

5. Pharmazeutische Zusammensetzung nach einem der Ansprüche 1 bis 4, wobei das lipidische Lösungsmittel oder das Gemisch von lipidischen Lösungsmitteln in einem Gewichtsanteil von 35 bis 55% vorliegt.

6. Pharmazeutische Zusammensetzung nach einem der Ansprüche 1 bis 5, wobei das oberflächenaktive Mittel aus einem einzigen oberflächenaktiven Mittel oder einem Gemisch von oberflächenaktiven Mitteln besteht.

7. Pharmazeutische Zusammensetzung nach einem der Ansprüche 1 bis 6, wobei das oberflächenaktive Mittel in einem Gewichtsanteil von 5 bis 15% vorliegt.

8. Pharmazeutische Zusammensetzung nach einem der Ansprüche 1 bis 7, wobei das Pyrazol-3-carboxamid-Derivat in einem Gewichtsanteil von 0,1 bis 6% vorliegt.

9. Pharmazeutische Zusammensetzung nach einem der Ansprüche 1 bis 8, wobei das oder die lipidische(n) Lösungsmittel ausgewählt ist/sind aus:
. Oleoyl-Macrogol-6-glyceriden (ungesättigten polyglycosylierten Glyceriden);
. Propylenglycolcaprylat/-caprat;
. Propylenglycolcaprylsäuremonoester;
. Glyceryloleat;
. Mono- und Diglycerid mittlerer Kettenlänge (Caprin-/Caprylsäure);
. Polyglycerinoleat;
. Capryl-/Caprinsäuretriglycerid.

10. Zusammensetzung nach einem der Ansprüche 1 bis 8, wobei das oder die oberflächenaktive(n) Mittel ausgewählt ist/sind aus:
. hydriertem Polyoxyethylen-35-Ricinöl;
. hydriertem Polyoxyethylen-40-Ricinöl;
. Polyoxyethylenpolysorbat;
. Sorbitanmonolaurat;
. Vitamin E/TPGS: Tocopherylpropylenglycol-1000-succinat;
. Polyethylenglycol-15-hydroxystearat.

11. Pharmazeutische Zusammensetzung nach Anspruch 2, wobei das oder die amphiphile(n) Co-Lösungsmittel ausgewählt ist/sind aus:
. Caprylocaproyl-Macrogol-8-glyceriden;
. Propylenglycolmonolaurat.

12. Pharmazeutische Zusammensetzung nach einem der Ansprüche 1 bis 11, die in Weichgelatinekapseln verabreichbar ist.

13. Pharmazeutische Zusammensetzung nach einem der Ansprüche 1 bis 11, die in versiegelten oder banderolierten Hartgelatinekapseln verabreichbar ist.

14. Pharmazeutische Zusammensetzung, die Folgendes enthält:
. Capryl-/Caprinsäuretriglycerid 45,9%
. hydriertes Polyoxyethylen-40- 12% Ricinöl
. Propylenglycolmonolaurat 21,5%
. Caprylocaproyl-Macrogol-8- 20% glyceride
. N-Piperidino-5-(4-bromphenyl)-1- 0,6% (2,4-dichlorphenyl)-4-ethylpyrazol-3-carboxamid

15. Pharmazeutische Zusammensetzung, die Folgendes enthält:
. Capryl-/Caprinsäuretriglycerid 41,5%
. hydriertes Polyoxyethylen-40- 12% Ricinöl
. Propylenglycolmonolaurat 21,5%
. Caprylocaproyl-Macrogol-8- 20% glyceride
. N-Piperidino-5-(4-bromphenyl)-1- 5% (2,4-dichlorphenyl)-4-ethylpyrazol-3-carboxamid

16. Pharmazeutische Zusammensetzung, die Folgendes enthält:
. Capryl-/Caprinsäuretriglycerid 41,5%
. hydriertes Polyoxyethylen-40- 12% Ricinöl
. Propylenglycolmonolaurat 21,5%
. Caprylocaproyl-Macrogol-8- 20% glyceride
. N-Piperidino-5-(4-chlorphenyl)-1- 5% (2,4-dichlorphenyl)-4-methylpyrazol-3-carboxamid
